(19) Europäisches Patentamt
European Patent Office
Office européen des brevets

(11) Veröffentlichungsnummer: **0 350 448**
**A1**

# (12) EUROPÄISCHE PATENTANMELDUNG

(21) Anmeldenummer: **89810491.4**

(22) Anmeldetag: **28.06.89**

(51) Int. Cl.[5]: **C 07 D 213/78**
C 07 D 213/81,
C 07 D 213/83,
C 07 D 239/28,
C 07 C 233/07,
C 07 C 257/18,
C 07 C 327/38,
C 07 D 401/04, A 61 K 31/44

(30) Priorität: **07.07.88 CH 2588/88**

(43) Veröffentlichungstag der Anmeldung:
**10.01.90 Patentblatt 90/02**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Stanek, Jaroslav, Dr.**
**Hangstrasse 9**
**CH-4144 Arlesheim (CH)**

**Caravatti, Giorgio, Dr.**
**Kurzelängeweg 30**
**CH-4123 Allschwil (CH)**

**Frei, Jörg, Dr.**
**Buechring 36**
**CH-4434 Hölstein (CH)**

**Capraro, Hans-Georg, Dr.**
**Habsburgerstrasse 60**
**CH-4310 Rheinfelden (CH)**

(54) **Biarylverbindungen.**

(57) Verbindungen der Formel I

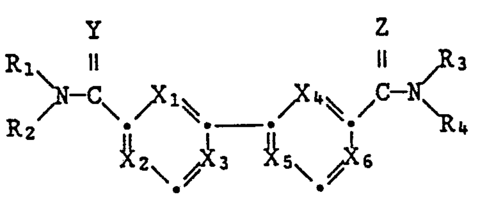

(I)

worin $X_1$, $X_2$, $X_3$, $X_4$, $X_5$, $X_6$, Y, Z und $R_1$-$R_4$ die in der Beschreibung angegebenen Bedeutungen haben, weisen wertvolle pharmazeutische Eigenschaften auf und sind insbesondere gegen Tumoren wirksam. Sie werden in an sich bekannter Weise hergestellt.

EP 0 350 448 A1

## Beschreibung

### Biarylverbindungen

Die Erfindung betrifft Verbindungen der Formel I

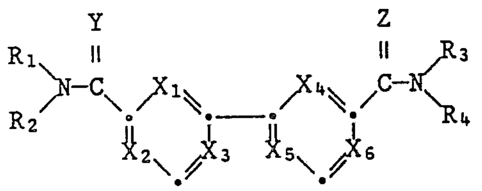

(I)

worin $X_1$, $X_2$ und $X_3$ unabhängig voneinander CH oder N bedeuten mit der Massgabe, dass mindestens eine der Gruppen $X_1$, $X_2$ oder $X_3$ für CH steht; $X_4$, $X_5$ und $X_6$ unabhängig voneinander CH oder N bedeuten mit der Massgabe, dass mindestens eine der Gruppen $X_4$, $X_5$ oder $X_6$ für CH steht; Y für $NR_5$, O oder S steht, Z für $NR_6$, O oder S steht, die Reste $R_2$, $R_4$, $R_5$, und $R_6$ unabhängig voneinander Wasserstoff oder Niederalkyl bedeuten und die Reste $R_1$ und $R_3$ unabhängig voneinander für Wasserstoff, Niederalkyl, Cycloalkyl, Arylniederalkyl, Aryl, freies oder funktionell abgewandeltes Carboxy, Hydroxy, verethertes oder verestertes Hydroxy oder unsubstituiertes oder mono- oder disubstituiertes Amino stehen; worin die Reste $R_1$ und $R_2$ zusammen auch Niederalkylen bedeuten können, worin die Reste $R_3$ und $R_4$ zusammen auch für Niederalkylen stehen können, worin die Reste $R_2$ und $R_5$ zusammen auch Niederalkylen bedeuten können, und worin die Reste $R_4$ und $R_6$ zusammen auch für Niederalkylen stehen können;
mit der Massgabe, dass Y für $NR_5$ oder S steht und Z $NR_6$ oder S bedeutet, wenn $X_3$ und $X_5$ für N und $X_1$, $X_2$, $X_4$ und $X_6$ für CH stehen;
und mit der Massgabe, dass Y $NR_5$ und Z $NR_6$ bedeuten, wenn
(a) $X_4$ und $X_5$ für N und $X_1$, $X_2$, $X_3$ und $X_6$ für CH stehen oder
(b) $X_1$ und $X_3$ für N und $X_2$, $X_4$, $X_5$ und $X_6$ für CH stehen;
Tautomere davon, und Salze davon, Verfahren zur Herstellung dieser Verbindungen, pharmazeutische Präparate, die diese Verbindungen enthalten, die Verwendung dieser Verbindungen zur therapeutischen Behandlung des menschlichen oder tierischen Körpers oder zur Herstellung pharmazeutischer Präparate.

Tautomere können z.B. dann auftreten, wenn Y für $NR_5$ steht und $R_1$ und/oder $R_2$ Wasserstoff bedeuten: Der entsprechende Amidinrest, in Formel I als $-C(=Y)-NR_1R_2$ dargestellt, kann dann z.B. auch in den tautomeren Formen $-C(-YH)=NR_1$ oder $-C(-YH)=NR_2$ vorliegen.
Ein weiteres Beispiel:
Steht Z für $NR_6$ und ist $R_3$ und/oder $R_4$ Wasserstoff, so kann die entsprechende Amidinstruktur, in Formel I als $-C(=Z)-NR_3R_4$ dargestellt, ebenso in den tautomeren Formen $-C(-ZH)=NR_3$ oder $-C(-ZH)=NR_4$ vorliegen. Dem Fachmann ist das Auftreten solcher und ähnlicher Tautomere geläufig. Alle diese Tautomere werden von der allgemeinen Formel I mit umfasst.

Die vor- und nachstehend verwendeten Allgemeinbegriffe haben im Rahmen der vorliegenden Anmeldung vorzugsweise die folgenden Bedeutungen:

Das Präfix "Nieder" bezeichnet einen Rest mit 1 bis 7 und insbesondere mit 1 bis 4 Kohlenstoffatomen.

Niederalkyl ist z.B. n-Propyl, Isopropyl, n-Butyl, Isobutyl, sek-Butyl, tert-Butyl, n-Pentyl, Neopentyl, n-Hexyl oder n-Heptyl, vorzugsweise Ethyl und vor allem Methyl.

Cycloalkyl enthält z.B. 3 bis 8, vorzugsweise 5 oder 6, Ringkohlenstoffatome und ist z.B. Cyclopropyl, Cyclopentyl, Cyclohexyl oder Cycloheptyl.

Arylniederalkyl ist vorzugsweise Phenylniederalkyl und insbesondere Benzyl.

Aryl ist z.B. Phenyl oder Naphthyl, wie 1- oder 2-Naphthyl. Die Phenyl-oder Naphthylreste können unsubstituiert oder substituiert sein. Aryl ist bevorzugt Phenyl, das unsubstituiert oder durch Niederalkyl, Nieder alkoxy, Halogen und/oder Nitro substituiert ist, und in erster Linie Phenyl.

Freies oder funktionell abgewandeltes Carboxy ist bevorzugt Cyano, ferner auch z.B. Carboxy, verestertes Carboxy, wie z.B. Niederalkoxycarbonyl, oder amidiertes Carboxy, wie z.B. Carbamoyl ($-CONH_2$), N-Niederalkylcarbamoyl oder N,N-Diniederalkylcarbamoyl.

Verethertes Hydroxy ist z.B. Niederalkoxy. Verestertes Hydroxy ist z.B. Niederalkanoyloxy. Monosubstituiertes Amino ist z.B. Niederalkylamino. Disubstituiertes Amino ist z.B. Diniederalkylamino, $C_4$-$C_6$-Alkylenamino, z.B. Piperidino, Oxa-$C_3$-$C_5$-alkylenamino, z.B. Morpholino, Thia-$C_3$-$C_5$-alkylenamino, z.B. Thiomorpholino, oder Aza-$C_3$-$C_5$-alkylenamino, welches unsubstituiert oder am Azastickstoff Niederalkyl-substituiert ist, z.B. Piperazino oder 4-Niederalkylpiperazino. Bevorzugt bedeutet disubstituiertes Amino Diniederalkylamino.

Niederalkylen gebildet aus den Gruppen $R_1$ und $R_2$ bzw. $R_3$ und $R_4$ ist bevorzugt $C_2$-$C_7$-Alkylen und insbesondere $C_4$-$C_5$-Alkylen, z.B. 1,4-Butylen oder 1,5-Pentylen.

Niederalkylen gebildet aus den Gruppen $R_2$ und $R_5$ bzw. $R_4$ und $R_6$ ist bevorzugt $C_2$-$C_5$-Alkylen und insbesondere $C_2$-$C_3$-Alkylen, z.B. 1,2-Ethylen oder 1,3-Propylen.

Halogen bedeutet z.B. Fluor oder Iod, insbesondere Brom und vor allem Chlor.

Salze von erfindungsgemässen Verbindungen sind in erster Linie pharmazeutisch verwendbare,

nicht-toxische Salze. Beispielsweise können Verbindungen der Formel I mit basischen Gruppen Säureadditionssalze, z.B. mit anorganischen Säuren, wie Salzsäure, Schwefelsäure oder Phosphorsäure, oder mit geeigneten organischen Carbon- oder Sulfonsäuren, z.B. Essigsäure, Fumarsäure oder Methansulfonsäure, oder z.B. mit Aminosäuren, wie Arginin oder Lysin, bilden. Bei Anwesenheit von mehreren basischen Gruppen können Mono- oder Polysalze gebildet werden. Ver bindungen der Formel I mit einer sauren Gruppe, z.B. Carboxy, und einer basischen Gruppe, z.B. Amino, können z.B. in Form von inneren Salzen, d.h. in zwitterionischer Form vorliegen, oder es kann ein Teil des Moleküls als inneres Salz, und ein anderer Teil als normales Salz vorliegen.

Zur Isolierung oder Reinigung können auch pharmazeutisch ungeeignete Salze, z.B. Pikrate oder Perchlorate, Verwendung finden. Zur therapeutischen Anwendung gelangen nur die pharmazeutisch verwendbaren, nicht-toxischen Salze, die deshalb bevorzugt sind.

Je nach den strukturellen Gegebenheiten können die Verbindungen der vorliegenden Erfindung in Form von Isomerengemischen oder von reinen Isomeren vorliegen.

Die erfindungsgemässen Verbindungen weisen wertvolle, insbesondere pharmakologisch verwendbare, Eigenschaften auf. Insbesondere haben sie eine starke, spezifische Hemmwirkung auf das Enzym S-Adenosylmethionindecarboxylase (SAMDC). SAMDC spielt als ein Schlüsselenzym eine wichtige Rolle bei der Polyaminsynthese, die in praktisch allen Zellen von Säugetieren, einschliesslich Menschen, abläuft. Durch SAMDC wird die Polyaminkonzentration in der Zelle reguliert. Eine Hemmung des Enzyms SAMDC hat eine Verringerung der Polyaminkonzentration zur Folge. Da eine Verringerung der Polyaminkonzentration eine Hemmung des Zellwachstums bewirkt, ist es möglich, durch Verabreichung von SAMDC-hemmenden Substanzen das Wachstum sowohl von eukaryontischen als auch prokaryontischen Zellen zu hemmen und sogar Zellen abzutöten oder das Einsetzen der Zelldifferenzierung zu hemmen.

Die Hemmung des Enzyms SAMDC kann z.B. mit der Methode von H.G. Williams-Ashman und A. Schenone, Biochem.Biophys.Res.Communs. 46, 288 (1972) nachgewiesen werden. Die Verbindungen der Erfindung weisen $IC_{50}$-Werte von minimal etwa 1 $\mu M$ auf.

Ein Vorteil der erfindungsgemässen Verbindungen besteht darin, dass sie nur in geringem Masse im Vergleich zu ihrer starken Hemmwirkung auf SAMDC die Diaminoxidase inhibieren und gut verträglich sind. Die Hemmung der Diaminoxidase ist nach J. Jaenne und D.R. Morris, Biochem. J. 218, 974 (1984) ungünstig, da sie zur Akkumulation von Putrescin und einer indirekten SAMDC-Aktivierung führen kann.

Daher sind die Verbindungen der Formel I z.B. nützlich zur Behandlung benigner und maligner Tumoren. Sie können Tumorregressionen bewirken und ferner die Verbreitung von Tumorzellen sowie das Wachstum von Mikrometastasen verhindern. Des weiteren können sie z.B. zur Behandlung von Protozoainfektionen, wie etwa Trypanosomiasis, Malaria oder durch Pneumocystis carinii verursachte Lungenentzündung, dienen.

Als selektive SAMDC-Hemmer können die Verbindungen der Formel I allein oder auch in Kombination mit anderen pharmakologisch wirksamen Substanzen angewendet werden. Zu denken ist z.B. an eine Kombination mit (a) Inhibitoren anderer Enzyme der Polyaminbiosynthese, z.B. Ornithindecarboxylasehemmern, (b) Inhibitoren der Proteinkinase C, (c) Inhibitoren der Tyrosinproteinkinase, (d) Cytokinen, (e) negativen Wachstumsregulatoren, (f) Aromatasehemmern, (g) Antiöstrogenen oder (h) klassischen zytostatischen Wirkstoffen.

Bevorzugt sind die Verbindungen der Formel I, worin $X_1$, $X_2$ und $X_3$ unabhängig voneinander CH oder N bedeuten mit der Massgabe, dass mindestens eine der Gruppen $X_1$, $X_2$ oder $X_3$ für CH steht; $X_4$, $X_5$ und $X_6$ unabhängig voneinander CH oder N bedeuten mit der Massgabe, dass mindestens eine der Gruppen $X_4$, $X_5$ oder $X_6$ für CH steht; Y für NH, O oder S steht, Z für NH, O oder S steht, die Reste $R_2$ und $R_4$ unabhängig voneinander Wasserstoff oder Niederalkyl bedeuten und die Reste $R_1$ und $R_3$ unabhängig voneinander für Wasserstoff, Niederalkyl, $C_3$-$C_8$-Cycloalkyl, Phenylniederalkyl, Phenyl, Carboxy, Hydroxy oder Amino stehen; worin die Reste $R_1$ und $R_2$ zusammen auch $C_2$-$C_7$-Alkylen bedeuten können, und worin die Reste $R_3$ und $R_4$ zusammen auch für $C_2$-$C_7$-Alkylen stehen können;
mit der Massgabe, dass Y und Z NH oder S bedeuten, wenn $X_3$ und $X_5$ für N und $X_1$, $X_2$, $X_4$ und $X_6$ für CH stehen;
und mit der Massgabe, dass Y und Z NH bedeuten, wenn
(a) $X_4$ und $X_5$ für N und $X_1$, $X_2$, $X_3$ und $X_6$ für CH stehen oder
(b) $X_1$ und $X_3$ für N und $X_2$, $X_4$, $X_5$ und $X_6$ für CH stehen; Tautomere davon, und Salze davon.

Besonders bevorzugt sind die Verbindungen der Formel I, worin $X_1$, $X_2$ und $X_3$ unabhängig voneinander CH oder N bedeuten mit der Massgabe, dass mindestens eine der Gruppen $X_1$, $X_2$ oder $X_3$ für CH steht; $X_4$, $X_5$ und $X_6$ unabhängig voneinander CH oder N bedeuten mit der Massgabe, dass mindestens eine der Gruppen $X_4$, $X_5$ oder $X_6$ für CH steht; Y für NH steht, Z für NH steht, die Reste $R_2$ und $R_4$ unabhängig voneinander Wasserstoff oder Niederalkyl bedeuten und die Reste $R_1$ und $R_3$ unabhängig voneinander für Wasserstoff, Niederalkyl, $C_3$-$C_8$-Cycloalkyl, Phenylniederalkyl, Phenyl, Carboxy, Hydroxy oder Amino stehen; worin die Reste $R_1$ und $R_2$ zusammen auch $C_2$-$C_7$-Alkylen bedeuten können, und worin die Reste $R_3$ und $R_4$ zusammen auch für $C_2$-$C_7$-Alkylen stehen können; Tautomere davon, und Salze davon.

Ganz besonders bevorzugt sind die Verbindungen der Formel I, worin (a) $X_1$ und $X_4$ für N stehen und $X_2$, $X_3$, $X_5$ und $X_6$ CH bedeuten oder (b) $X_2$ und $X_6$ für N stehen und $X_1$, $X_3$, $X_4$ und $X_5$ CH bedeuten; Y für NH oder O steht, Z für NH oder O steht, die Reste $R_2$ und $R_4$ Wasserstoff bedeuten und die Reste $R_1$ und $R_3$ für Wasserstoff, Niederalkyl, Hydroxy oder Amino stehen, Tautomere davon, und pharmazeutisch verwendbare Salze davon.

In erster Linie bevorzugt sind die Verbindungen der Formel I, worin (a) $X_1$ und $X_4$ für N stehen und $X_2$, $X_3$, $X_5$ und $X_6$ CH bedeuten oder (b) $X_2$ und $X_6$ für N stehen und $X_1$, $X_3$, $X_4$ und $X_5$ CH bedeuten; Y für NH steht, Z für NH steht, die Reste $R_2$ und $R_4$ Wasserstoff bedeuten und die Reste $R_1$ und $R_3$ für Wasserstoff, Niederalkyl, $C_5$-$C_6$-Cycloalkyl oder Hydroxy stehen, Tautomere davon, und pharmazeutisch verwendbare Salze davon.

Als Untergruppen aus einer Gruppe von Verbindungen der Formel I sind jeweils hervorzuheben: (a) Verbindungen der Formel I, worin die Gruppen $-C(=Y)-NR_1R_2$ und $-C(=Z)-NR_3R_4$ identisch sind; (b) Verbindungen der Formel I, worin die Gruppen

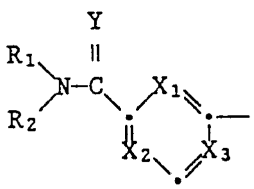 und 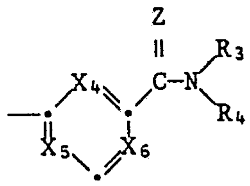

identisch sind; (c) Verbindungen der Formel I, worin die Symbole $X_1$-$X_6$ die folgenden Bedeutungen haben:

(1) $X_1 = X_4 = N$ und $X_2 = X_3 = X_5 = X_6 = CH$ oder
(2) $X_2 = X_6 = N$ und $X_1 = X_3 = X_4 = X_5 = CH$ oder
(3) $X_3 = X_5 = N$ und $X_1 = X_2 = X_4 = X_6 = CH$ oder
(4) $X_1 = X_2 = X_3 = X_4 = X_5 = X_6 = CH$ oder
(5) $X_4 = N$ und $X_1 = X_2 = X_3 = X_5 = X_6 = CH$ oder
(6) $X_5 = N$ und $X_1 = X_2 = X_3 = X_4 = X_6 = CH$ oder
(7) $X_6 = N$ und $X_1 = X_2 = X_3 = X_4 = X_5 = CH$ oder
(8) $X_5 = X_6 = N$ und $X_1 = X_2 = X_3 = X_4 = CH$ oder
(9) $X_4 = X_6 = N$ und $X_1 = X_2 = X_3 = X_5 = CH$; (d) Verbindungen der Formel I, worin die Symbole $X_1$-$X_6$ die unter (c) angegebenen Bedeutungen (1), (2), (3) oder (4) haben; (e) Verbindungen der Formel I, worin $R_1$, $R_2$, $R_3$ und $R_4$ Wasserstoff bedeuten; (f) Verbindungen der Formel I, worin mindestens eine der Gruppen $X_1$-$X_6$ für N steht.

Die Erfindung betrifft vor allem die in den Beispielen beschriebenen spezifischen Verbindungen und pharmazeutisch anwendbare Salze davon.

Die Verbindungen der Formel I können in an sich bekannter Weise hergestellt werden, indem man z.B. in einer Verbindung der Formel II

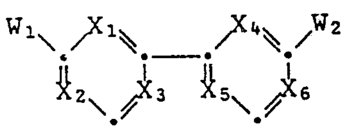

(II)

worin $W_1$ und $W_2$ jeweils einen in die Gruppe $-C(=Y)NR_1R_2$ bzw. $-C(=Z)NR_3R_4$ überführbaren Rest bedeuten, die Reste $W_1$ und $W_2$ in die Gruppe $-C(=Y)NR_1R_2$ bzw. $-C(=Z)NR_3R_4$ überführt; wobei die Symbole $X_1$-$X_6$, Y, Z und $R_1$-$R_4$ die unter Formel I angegebene Bedeutung haben; und/oder eine erhaltene Verbindung der Formel I in eine andere Verbindung der Formel I umwandelt, und/oder ein erhaltenes Salz in die freie Verbindung oder in ein anderes Salz umwandelt, und/oder eine erhaltene freie Verbindung der Formel I mit salzbildenden Eigenschaften in ein Salz umwandelt.

In der folgenden näheren Beschreibung des Verfahrens haben die Symbole $X_1$-$X_6$, Y, Z und $R_1$-$R_6$ jeweils die unter Formel I angegebene Bedeutung, sofern nichts anderes angegeben ist.

In den Zwischenprodukten der Formel II bedeuten $W_1$ und $W_2$ z.B. freies oder funktionell abgewandeltes Carboxy, insbesondere Halogencarbonyl, Carbamoyl, N-Niederalkylcarbamoyl, Cyan, einen Imino-niederalkylester [$-C(=NH)-OAlk$ (Alk $\hat{=}$ Niederalkyl)] oder Imino-niederalkylthiolester [$-C(=NH)-SAlk$].

Die Gruppen $W_1$ und/oder $W_2$ in einer Verbindung der Formel II können bei der Herstellung von (Mono- oder Di-)Amidinen der Formel I (Y $\hat{=}$ $NR_5$ und/oder Z $\hat{=}$ $NR_6$) z.B. bedeuten: ein Säureadditionssalz eines Imino-niederalkylesters ($\hat{=}$ Imino-niederalkylether) oder Imino-niederalkylthiolesters, z.B. $-C(=NH)-OC_2H_5 \bullet HCl$ bzw. $-C(=NH)-SC_2H_5 \bullet HI$, Cyano oder N-Niederalkylcarbamoyl.

Durch die Umsetzung von (Mono- oder Di-)Imino-niederalkylestern bzw. (Mono- oder Di-)Imino-niederalkylthiolestern der Formel II (als Salze) mit Ammoniak oder primären bzw. sekundären Aminen erhält man die unsubstituierten bzw. mono- oder disubstituierten (Mono- oder Di-)-Amidine der Formel I. (Mono- oder Di-)Cyanoverbindungen der Formel II können z.B. durch Umsetzung mit einem Alkalimetallamid, z.B. $KNH_2$, oder durch Reaktion mit einem primären oder sekundären (Di-)niederalkylammoniumhalogenid, z.B. $H_3CH_3$ $Cl^{\ominus}$, in ein gegebenenfalls mono- oder disubstituiertes (Mono- oder Di-)Amidin der Formel I überführt werden.

Verbindungen der Formel II, worin $W_1$ und/oder $W_2$ N-Niederalkylcarbamoyl bedeuten, lassen sich z.B. durch Umsetzung mit $POCl_3$ oder $PCl_5$ in die entsprechenden Imidsäurechloride [$-C(=NH-Alk)-Cl$] überführen, welche nach Reaktion mit Ammoniak beziehungsweise einem primären oder sekun dären Amin substituierte (Mono- oder Di-)Amidine der Formel I ergeben [vgl. Chem. Abstr. 81, 91185a (1974)]

Verbindungen der Formel I, worin die Reste $R_2$ und $R_5$ zusammen und/oder die Reste $R_4$ und $R_6$ zusammen

Niederalkylen bedeuten, lassen sich z.B. dadurch herstellen, dass man eine Verbindung der Formel II, worin $W_1$ und/oder $W_2$ Cyano bedeuten, mit einem α, ω-Diaminoniederalkan, z.B. 1,2-Diaminoethan, - bevorzugt in Gegenwart von katalytischen Mengen Schwefelkohlenstoff - umsetzt.

Die Gruppen $W_1$ und/oder $W_2$ in einer Verbindung der Formel II können bei der Herstellung von (Mono- oder Di-)Carbamoylverbindungen der Formel I ($Y \hat{=} O$ und/oder $Z \hat{=} O$) z.B. bedeuten: Carboxy, Halocarbonyl (z.B. -COCl), Niederalkoxycarbonyl oder Cyano. Die Bildung von gegebenenfalls mono- oder disubstituierten (Mono- oder Di-)Carbamoylverbindungen der Formel I aus entsprechenden Zwischenprodukten der Formel II, worin $W_1$ und/oder $W_2$ Carboxy, Halocarbonyl oder Niederalkoxycarbonyl bedeuten, durch Umsetzung mit Ammoniak bzw. primären oder sekundären Aminen ist an sich bekannt. Zwischenprodukte der Formel II, worin $W_1$ und/oder $W_2$ Cyano bedeuten, können z.B. durch partielle Hydrolyse, im Sinne einer Graf-Ritter-Reaktion, oder über Imino-niederalkylester-Salze in gegebenenfalls mono- oder disubstituierte (Mono- oder Di-)Carbamoylverbindungen der Formel I überführt werden. Die Bedingungen bei der Hydrolyse der Cyano-Zwischenprodukte können so gewählt werden, dass die Reaktion auf der Stufe des Amids abgebrochen wird. Zu diesem Zweck ist insbesondere die Hydrolyse mit Säuren geeignet, wobei z.B. 80%ige Schwefelsäure (unter Erwärmen), Polyphosphorsäure (bei 100-150° C), Bromwasserstoff/Eisessig (Raumtemperatur, Ameisensäure oder ohne Lösungsmittel), HCl-Gas in etherischer Lösung gefolgt von der Zugabe von Wasser oder wässriger Salzsäure, oder Borhalogenide in Frage kommen.

Mit Hilfe der Graf-Ritter-Reaktion gelingt auch die Herstellung N-substituierter Amide aus (Mono- oder Di-)Nitrilen der Formel II. Hierzu setzt man die (Mono- oder Di-)Nitrile in Gegenwart einer starken Säure, vornehmlich 85-90%iger Schwefelsäure, oder auch Polyphosphor säure, Ameisensäure, Bortrifluorid oder anderen Lewis-Säuren, nicht jedoch Aluminiumchlorid, mit Verbindungen um, die in dem sauren Medium Carbeniumionen bilden können, also z.B. mit Olefinen, wie Propylen, oder Alkoholen, wie Ethanol.

Die (Mono- oder Di-)Imino-niederalkylester erhält man z.B. durch säurekatalysierte Anlagerung von Alkoholen an die (Mono- oder Di-)Nitrile der Formel II. Diese Anlagerung kann gleichfalls durch Basen, z.B. Alkoholate wie Natriummethoxid, katalysiert werden. Aus den (Mono-oder Di-)Imino-niederalkylestern erhält man die (Mono- oder Di-)Amide im Sinne einer Pinner-Spaltung durch thermischen Zerfall der Iminoester-Salze bei Temperaturen oberhalb von etwa 80° C.

Andererseits können die (Mono- oder Di-)Imino-niederalkylester z.B. auch aus (Mono- oder Di-)Carbamoylverbindungen der Formel II durch Umsetzung mit Triniederalkyloxonium-tetrafluoroborat, insbesondere $^{\oplus}O(C_2H_5)_3\ BF_4^{\ominus}$ ("Meerweinsalz"), hergestellt werden.

Die (Mono- oder Di-)Imino-niederalkylthiolester werden z.B. durch S-Alkylierung der entsprechenden (Mono- oder Di-)Thiocarbamoylverbindungen (s.u.) hergestellt; zur Alkylierung können z.B. Niederalkylhalogenide oder -tosylate verwendet werden.

Die Gruppen $W_1$ und/oder $W_2$ in einer Verbindung der Formel II können bei der Herstellung von (Mono- oder Di-)Thiocarbamoylverbindungen der Formel I ($Y \hat{=} S$ und/oder $Z \hat{=} S$) z.B. bedeuten: Carbamoyl, Niederalkyl- oder Diniederalkylcarbamoyl (in diesen Fällen entspricht die Verbindung der Formel II einer Verbindung der Formel I), Cyano oder Halogencarbonyl. Die Bildung von gegebenenfalls mono- oder disubstituierten (Mono- oder Di-)Thiocarbamoylverbindungen der Formel I durch Reaktion entsprechender Zwischenprodukte der Formel II, worin $W_1$ und/oder $W_2$ z.B. wie oben angegeben definiert ist, mit Schwefel-ein-führenden Agentien ist an sich bekannt [vgl. z.B. Chem. Reviews 61, 45-86 (1961)]. Zwischenprodukte der Formel II, worin $W_1$ und/oder $W_2$ Carbamoyl, Niederalkyl- oder Diniederalkylcarbamoyl bedeuten, können z.B. durch Umsetzung mit Phosphorpentasulfid ($P_4S_{10}$ oder Aluminium trisulfid ($Al_2S_3$) oder insbesondere dem Lawesson-Reagens [2,4-Bis-(4-methoxyphenyl)-2,4-dithioxo-1,3,2,4-dithiadiphosphetan] in gegebenenfalls mono- oder disubstituierte (Mono- oder Di-)Thiocarbamoylverbindungen der Formel I überführt werden. Zwischenprodukte der Formel II, worin $W_1$ und/oder $W_2$ Cyano bedeuten, lassen sich z.B. durch Reaktion mit Ammoniak und Schwefelwasserstoff, und solche worin $W_1$ und/oder $W_2$ Halogencarbonyl bedeuten, z.B. durch Umsetzung mit Phosphorpentachlorid, Schwefelwasserstoff und Ammoniak in (Mono- oder Di-)Thiocarbamoylverbindungen der Formel I überführen.

Verbindungen der Formel II, worin $W_1$ und $W_2$ Carboxy bedeuten, werden z.B. hergestellt, indem man eine Verbindung der Formel III

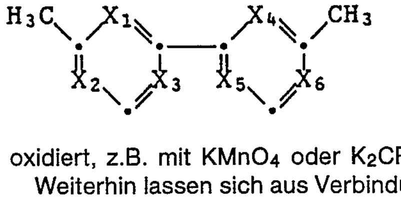

(III)

oxidiert, z.B. mit $KMnO_4$ oder $K_2CR_2O_7$.

Weiterhin lassen sich aus Verbindungen der Formel III auch z.B. Verbindungen der Formel II, worin $W_1$ und $W_2$ Cyano bedeuten, herstellen, indem man erstere z.B. analog Chem. Pharm. Bull. 25, 1821 (1977) [$\hat{=}$ C.A. 88, 121089 m (1978)] mit Ethylnitrit in Gegenwart von Alkalimetallamiden in flüssigem Ammoniak zu den entsprechenden Dialdoximen nitrosiert und diese zusammen mit $POCl_3$ erhitzt.

Verbindungen der Formel II, worin $W_1$ und $W_2$ Cyano bedeuten, werden ferner z.B. dadurch hergestellt, dass man eine Verbindung der Formel IV

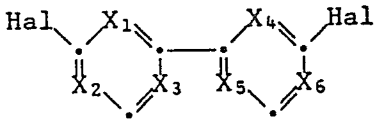

(IV)

worin Hal Halogen bedeutet, mit Cyanierungsmitteln, z.B. Natriumcyanid oder Kaliumcyanid - gegebenenfalls unter Katalyse durch $Pd[P(C_6H_5)_3]_4$ oder Phasentransferkatalyse, z.B. mit 18-Krone-6-Ether, - oder mit Kupfer(I)cyanid insbesondere in Pyridin oder Dimethylformamid [vgl. Chem. Rev. 87, 779 (1987)], umsetzt.

Andererseits können die Halogengruppen in einer Verbindung der Formel IV auch z.B. durch Umsetzung mit Trimethylamin in Trimethylammoniumhalogenid-Reste umgewandelt werden. Letztere lassen sich dann z.B. durch Behandlung mit Natriumcyanid im wässrigen Medium in Cyanogruppen überführen, wobei wiederum Verbindungen der Formel II, worin $W_1$ und $W_2$ Cyano bedeuten, erhalten werden.

Weiterhin lassen sich aus Verbindungen der Formel IV auch z.B. Verbindungen der Formel II, worin $W_1$ und $W_2$ Carboxy bedeuten, herstellen, indem man erstere dimetalliert und dann z.B. mit $CO_2$ umsetzt. Die Metallierung kann z.B. mit Lithiierungsmitteln, z.B. n-Butyllithium, durchgeführt werden und führt dann zu Di-(Li)-Zwischenprodukten. Wird mit Lithiierungsmitteln und Kupfer(I)salzen metalliert, so erhält man Dicuprate, z.B. Di-(CuLi), als Zwischenstufen. Des weiteren kann man z.B. mit Magnesium metallieren und erhält dann Dimagnesiumhalogenide, Di-(MgHal), als Zwischenprodukte.

Verbindungen der Formel II, worin $W_1$ und $W_2$ Cyano bedeuten, können z.B. auch hergestellt werden, indem man eine Verbindung der Formel V

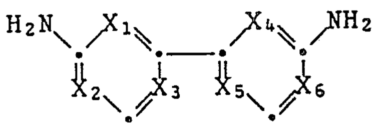

(V)

zweifach diazotiert, z.B. mit Natriumnitrit, und dann mit z.B. Kupfer(I)cyanid umsetzt (Sandmeyer-Reaktion).

Verbindungen der Formel II, worin $W_1$ und $W_2$ Cyano bedeuten, werden ferner z.B. dadurch hergestellt, dass man eine Verbindung der Formel VI

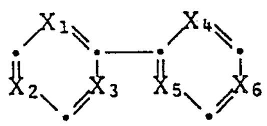

(VI)

worin mindestens einer der Reste $X_1$ oder $X_2$ und mindestens einer der Reste $X_4$ oder $X_6$ für N steht, zu einem Di-N-oxid oxidiert - z.B. mit meta-Chlorperbenzoesäure oder $H_2O_2$ -, dieses dimethyliert - z.B. mit Dimethylsulfat - und das erhaltene N,N'-Dimethoxyderivat mit z.B. Natriumcyanid umsetzt. Anstatt das erwähnte Di-N-oxid zu dimethylieren und mit z.B. NaCN umzusetzen, kann man es z.B. auch direkt mit Trimethylsilylcyanid reagieren lassen (vgl. Synthesis 1984, 681).

Verbindungen der Formel II, worin $W_1$ und $W_2$ Cyano bedeuten, bzw. Verbindungen der Formel III, IV und VI werden z.B. hergestellt, indem man eine Verbindung der Formel VIIa

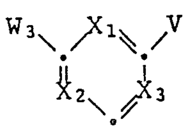

(VIIa)

mit einer Verbindung der Formel VIIb

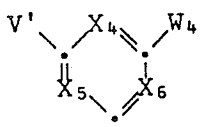

(VIIb)

umsetzt. In den Verbindungen der Formel VIIa und VIIb bedeuten $W_3$ und $W_4$ jeweils Cyano bzw. Methyl, Halogen oder Wasserstoff und V und V′ stehen für funktionelle Gruppen, die zur Verknüpfung von (Hetero-)-Arylen zu Bi-(hetero-)arylen geeignet sind. Geeignete Gruppen V und V′ sind z.B. (a) Magnesiumhalogenid -MgHal und Hal (Hal $\hat{=}$ Halogen), (b) Lithium -Li und Hal oder (c) Cuprate, z.B. -CuLi, und Hal.

Ferner ist (d) die Kupplung von zwei (gleichen oder verschiedenen) Lithiumderivaten (V $\hat{=}$ V′ $\hat{=}$ Li) in Gegenwart von z.B. $CuCl_2/O_2$ oder $[CuI \bullet P(n\text{-}C_4H_9)_3]_4/O_2$ [vgl. J. Organomet. Chem. 56, 53 (1973)] zu erwähnen.

Eine weitere Möglichkeit zur Verknüpfung besteht (e) in der Kupplung einer Verbindung der Formel VIIa, worin V $\hat{=}$ Li, mit einer (N-heterocyclischen)Verbindung der Formel VIIb, worin V′ $\hat{=}$ H, vgl. Chem. Ber. 113,

2739 (1980).

Verbindungen der Formel V können z.B. durch Reduktion der entsprechenden Dinitroverbindungen, z.B. durch Hydrierung oder mit $Sn(II)Cl_2$, hergestellt werden. Weiterhin lassen sich Verbindungen der Formel V z.B. auch aus den entsprechenden Verbindungen der Formel IV herstellen, indem letztere mit Alkalimetallamid, z.B. Natrium- oder Kaliumamid, umgesetzt werden.

Die Verbindungen der Formel II lassen sich ferner durch Totalsynthese herstellen. Dabei geht man normalerweise von einem monozyklischen Derivat aus und baut den zweiten - bevorzugt N-heterozyklischen - Ring auf. Beispielsweise kann eine Verbindung der Formel II, worin $W_1$ und $W_2$ Cyano bedeuten, $X_1$, $X_2$, $X_3$ und $X_4$ für CH stehen und $X_5$ und $X_6$ N bedeuten [$\hat{=}$ 4-Cyano-6-(3-cyanophenyl)-pyrimidin], über folgende Syntheseschritte erhalten werden:

(a) 3-Cyano-benzoesäurechlorid + Malonsäuremonoethylester → 3-Cyano phenyl-3-on-propionsäureethylester

(b) Produkt von (a) + Thioharnstoff/Entschwefelung mit Raney-Nickel → 4-Hydroxy-6-(3-cyanophenyl)-pyrimidin

(c, d) Produkt von (b) + $POCl_3$, dann + Cu(I)CN → 4-Cyano-6-(3-cyano-phenyl)-pyrimidin ($\hat{=}$ gewünschte Verbindung der Formel II).

Verfahrensgemäss erhältliche freie Verbindungen der Formel I mit salzbildenden Eigenschaften können in an sich bekannter Weise in ihre Salze übergeführt werden, Verbindungen mit basischen Eigenschaften durch Behandeln mit Säuren oder geeigneten Derivaten davon, Verbindungen mit sauren Eigenschaften durch Behandeln mit Basen oder geeigneten Derivaten davon.

Erfindungsgemäss erhältliche Gemische von Isomeren können in an sich bekannter Weise in die einzelnen Isomeren aufgetrennt werden, Racemate z.B. durch Bilden von Salzen mit optisch reinen salzbildenden Reagentien und Auftrennen des so erhältlichen Diastereomerengemisches, z.B. mittels fraktionierter Kristallisation.

Die oben angeführten Reaktionen können unter an sich bekannten Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln oder neutralisierenden Agentien, je nach Art der Reaktion und/oder der Reaktionsteilnehmer bei erniedrigter, normaler oder erhöhter Temperatur, z.B. im Temperaturbereich von etwa -70°C bis etwa 190°C, vorzugsweise von etwa -20°C bis etwa 150°C, z.B. beim Siedepunkt des verwendeten Lösungsmittels, unter atmosphärischem Druck oder in einem geschlossenen Gefäss, gegebenenfalls unter Druck, und/oder in einer inerten Atmosphäre, z.B. unter einer Stickstoffatmosphäre.

Im Verfahren der vorliegenden Erfindung werden vorzugsweise solche Ausgangsstoffe eingesetzt, die zu den eingangs als besonders wertvoll beschriebenen Verbindungen führen.

Die Erfindung betrifft auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt, oder bei denen ein Ausgangsstoff unter den Reaktionsbedingungen gebildet oder in Form eines Derivates, z.B. eines Salzes davon, verwendet wird.

Die vorliegende Erfindung betrifft ebenfalls pharmazeutische Präparate, die als Wirkstoff eine der pharmakologisch wirksamen Verbindungen der Formel I enthalten. Besonders bevorzugt sind Präparate zur enteralen, insbesondere oralen, sowie zur parenteralen Verabreichung. Die Präparate enthalten den Wirkstoff allein oder vorzugsweise zusammen mit einem pharmazeutisch anwendbaren Trägermaterial. Die Dosierung des Wirkstoffs hängt von der zu behandelnden Krankheit, sowie von Spezies, deren Alter, Gewicht und individuellem Zustand, sowie von der Applikationsweise ab.

Die pharmazeutischen Präparate enthalten von etwa 5 % bis etwa 95 % des Wirkstoffs, wobei einzeldosierte Applikationsformen vorzugsweise von etwa 20 % bis etwa 90 % und nicht-einzeldosierte Applikationsformen vorzugsweise etwa 5 % bis etwa 20 % Wirkstoff aufweisen. Dosiseinheitsformen, wie Dragées, Tabletten oder Kapseln, enthalten von etwa 0,05 g bis etwa 1,0 g des Wirkstoffs.

Die pharmazeutischen Präparate der vorliegenden Erfindung werden in an sich bekannter Weise, z.B. mittels konventioneller Misch-, Granulier-, Dragier-, Lösungs-, oder Lyophilisierungsverfahren hergestellt. so kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls durch Zugabe von zusätzlichen Hilfsstoffen, zu Tabletten oder Dragées-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B. Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Alginsäure oder ein Salz davon, wie Natriumalginat. Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol, oder Derivate davon.

Dragées-Kerne können mit geeigneten gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Oral anwendbare pharmazeutische Zusammensetzungen sind ebenfalls Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten flüssigen Hilfsstoffen, wie fetten Oelen, Paraffinöl oder flüssigen Polyethylenglykolen, gelöst oder suspendiert, wobei ebenfalls Stabilisatoren zugefügt sein können.

Weitere orale Applikationsformen sind z.B. in üblicher Weise bereitete Sirups, die den Wirkstoff z.B. in suspendierter Form und in einer Konzentration von ca. 5 % bis 20 %, vorzugsweise ca. 10 % oder in einer ähnlichen Konzentration, die z.B. beim Abmessen von 5 oder 10 ml eine geeignete Einzeldosis ergibt, enthalten. Ferner kommen z.B. auch pulverförmige oder flüssige Konzentrate zur Bereitung von Shakes, z.B. in Milch, in Betracht. Solche Konzentrate können auch in Einzeldosismengen abgepackt sein.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole.

Zu parenteralen Verabreichung eignen sich in erster Linie wässrige Lösungen eines Wirkstoffes in wasserlöslicher Form, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Lösungen, wie sie z.B. für die parenterale Verabreichung verwendet werden, können auch als Infusionslösungen angewandt werden.

Die Erfindung betrifft ebenfalls ein Verfahren zur Behandlung der oben genannten Krankheitszustände. Die Verbindungen der vorliegenden Erfindung können prophylaktisch oder therapeutisch verabreicht werden, wobei man sie vorzugsweise in Form von pharmazeutischen Präparaten verwendet. Dabei wird bei einem Körpergewicht von etwa 70 kg eine tägliche Dosis von etwa 0,1 g bis etwa 10 g, vorzugsweise von etwa 0,5 g bis etwa 5 g einer Verbindung der vorliegenden Erfindung verabreicht.

Die nachfolgenden Beispiele illustrieren die vorliegende Erfindung; Temperaturen werden in Grad Celsius angegeben.

Beispiel 1: 2,2′-Diamidino-4,4′-bipyridin-dihydrochlorid

0,028 g (0,0012 g-Atom) Natrium werden unter Stickstoff in 15 ml abs. Methanol gelöst. Zu dieser Lösung werden 2,5 g (0,0124 Mol) 2,2′-Dycyano-4,4′-bipyridin [Experientia 30, 843 (1974)] gegeben, und das resultierende Gemisch wird 2 Tage bei Raumtemperatur gerührt. Nach Zugabe von 1,53 g (0,0286 Mol) Ammoniumchlorid, 10 ml abs. Methanol und 20 ml gesättigter ethanolischer Ammoniaklösung wird das Reaktionsgemisch 1 h auf ca. 70° erwärmt und anschliessend über Nacht bei Raumtemperatur stehengelassen. Die Suspension wird eingedampft, in Wasser gelöst und mit Methylenchlorid gewaschen. Die wässrige Phase wird zur Trockne eingedampft und der Rückstand zweimal aus verdünnter Salzsäure umkristallisiert. Man erhält das Monohydrat der Titelverbindung, Smp. >280°.

Beispiel 2: 6,6′-Diamidino-2,2′-bipyridin-dihydrochlorid

Ausgehend von 15 g 6,6′-Dicyano-2,2′-bipyridin wird analog Beispiel 1 die Titelverbindung, Smp. >300°, hergestellt.

Die Ausgangsverbindungen werden wie folgt hergestellt:

(a) 6,6′-Dicyano-2,2′-bipyridin

11,4 ml (0,12 Mol) Dimethylsulfat werden bei 75° mit 11,1 g (0,06 Mol) 2,2′-Bipyridin-di-N-oxid [J. Heterocycl. Chem. 11, 299 (1974)] versetzt. Es entsteht eine dicke Suspension, welcher nochmals 11,4 ml (0,12 Mol) Dimethylsulfat zugesetzt werden. Danach wird noch 15 min auf 80° erwärmt, abgekühlt und am Hochvakuum eingedampft. Der Rückstand wird in 30 ml Wasser gelöst und langsam in eine eisgekühlte Lösung von 17,4 g (0,33 Mol) Natriumcyanid in 66 ml Wasser getropft. Das 6,6′-Dicyano-2,2′-bipyridin kristallisiert schon während der Zugabe des Natriumcyanids aus. Nach beendeter Zugabe wird mit 60 ml Wasser verdünnt, abfiltriert, mit Wasser gewaschen und das Produkt am Hochvakuum getrocknet. Das Rohprodukt wird mit wenig Ethanol verrührt, abfiltriert und erneut getrocknet; Smp. 255-258°.

Beispiel 3: 2,2′-Dicarbamoyl-4,4′-bipyridin-dihydrochlorid

5,5 g (0,025 Mol) 2,2′-Dicyano-4,4′-bipyridin werden in 300 ml Chloroform und 32,5 ml (0,56 Mol) abs. Ethanol gelöst und die Lösung bei Raumtemperatur mit trockenem Salzsäuregas gesättigt. Das Reaktionsgemisch wird anschliessend 24 h bei Raumptemperatur gerührt. Die Suspension wird filtriert und der Festkörper am

Hochvakuum getrocknet. Man erhält die Titelverbindung, Smp. 280° (Zers.).

Beispiel 4: 4,4'-Diamidino-2,2'-bipyridin-dihydrochlorid

Eine Suspension von 1,2 g (0,0058 Mol) 4,4'-Dicyano-2,2'-bipyridin in 48 ml abs. Methanol wird mit 0,5 ml 1,1 N Natriummethoxid in abs. Methanol versetzt und 5 h am Rückfluss gekocht. Zu der noch warmen Lösung werden danach 0,05 ml Eisessig, 0,72 g (0,0134 Mol) Ammoniumchlorid und 10 ml 4,6 N methanolische Ammoniaklösung gegeben, und man kocht eine weitere Stunde nach. Das abgekühlte Reaktionsgemisch wird eingedampft, in 60 ml 0,1 N Salzsäure aufgenommen, filtriert und auf eine Säule von 250 ml Amberlite® ER 180 Adsorberharz aufgetragen. Man wäscht mit dest. Wasser und sammelt 5 ml-Fraktionen. Die Fraktionen 14-21 werden vereinigt, eingedampft und der Rückstand aus wenig Wasser kristallisiert. Man erhält so die Titelverbindung; Smp. >280°.

Die Ausgangsverbindung wird wie folgt hergestellt:

Eine Suspension von 4,73 g (0,0195 Mol) 4,4'-Dicarbamoyl-2,2'-bipyridin [J. Am. Chem. Soc. 80, 2745 (1958)] und 5,67 g (0,0476 Mol) Thionylchlorid in 30 ml Pyridin wird 16 h bei 100° gerührt. Nach dem Abkühlen wird das Reaktionsgemisch mit 100 ml Methylenchlorid verdünnt, filtriert und zur Trockne eingedampft. Der Rückstand wird anschliessend mit Methylenchlorid über Kieselgel chromatographiert. Man erhält so das 4,4'-Dicyano-2,2'-bipyridin als weisse Kristalle vom Smp. 238-240°.

Beispiel 5: 3,3'-Diamidino-biphenyl-dihydrochlorid

1,0 g (3,6 mMol) 3,3'-Dithiocarbamoyl-biphenyl (vgl. Beispiel 14) werden unter Stickstoff in 45 ml trockenem Methylenchlorid suspendiert und bei Raumtemperatur mit 1,4 g (7,3 mMol) Triethyloxonium-tetrafluoroborat versetzt. Nach 2 h gibt man der Reaktionslösung ein Gemisch von 348 mg (2,5 mMol) Kaliumcarbonat und 0,35 ml Wasser zu, rührt kurz nach, filtriert und wäscht das Filtrat mit Eiswasser. Die organische Phase wird über Natriumsulfat getrocknet und eingedampft. Der rohe Di-thioiminoether, 530 mg (1,6 mMol), wird in 5 ml abs. Ethanol gelöst, mit 0,203 g (3,8 mMol) Ammoniumchlorid versetzt und 6 h unter Rückfluss erhitzt. Nach dem Abkühlen wird filtriert, dem Filtrat wenig ethanolische Salzsäure zugesetzt und eingedampft. Die Titelverbindung wird durch Chromatographie an Amberlite® XAD 1180 (Wasser als Eluiermittel) gereinigt; Smp. 230-235°.

Beispiel 6: 2-Amidino-6-(3-amidinophenyl)-pyridin

Ausgehend von 2,0 g 2-Cyano-6-(3-cyanophenyl)-pyridin wird analog Beispiel 1 die Titelverbindung hergestellt.

Die Ausgangsverbindungen werden wie folgt hergestellt:

(a) 2-(3-Cyanophenyl)-pyridin-N-oxid

Ausgehend von 2-(3-cyanophenyl)-pyridin wird durch Oxidation mit $H_2O_2$ in Eisessig [J. Heterocycl. Chem. 11, 299 (1974)] die Titelverbindung hergestellt.

(b) 2-Cyano-6-(3-cyanophenyl)-pyridin

Ausgehend von 2-(3-cyanophenyl)-pyridin-N-oxid wird analog Beispiel 2a die Titelverbindung hergestellt.

Beispiel 7: 4-Amidino-2-(3-amidinophenyl)-pyridin

Ausgehend von 2,0 g 4-Cyano-2-(3-cyanophenyl)-pyridin wird analog Beispiel 1 die Titelverbindung hergestellt.

Die Ausgangsverbindungen werden wie folgt hergestellt:

(a) 4-Chlor-2-(3-bromphenyl)-pyridin

Ausgehend von 3-Bromphenyllithium, 4-Chlorpyridin und Chlorameisensäurephenylester (zur Acylierung des Pyridinstickstoffs) wird analog J. Org. Chem. 50, 4410 (1985) die Titelverbindung hergestellt. Dabei entsteht intermediär ein entsprechendes 1-Acyl-1,2-dihydropyridin, welches durch Umsetzung mit o-Chloranil ($\hat{=}$ 3,4,5,6-Tetrachlor-1,2-benzochinon) zur Titelverbindung aromatisiert wird.

(b) 4-Cyano-2-(3-cyanophenyl)-pyridin

Ausgehend von 4-Chlor-2-(3-bromphenyl)-pyridin wird durch Reaktion mit Kupfercyanid in siedendem N,N-Dimethylformamid die Titelverbindung hergestellt.

Beispiel 8: 2-Amidino-4-(3-amidinophenyl)-pyridin

Ausgehend von 2-Cyano-4-(3-cyanophenyl)-pyridin wird analog Beispiel 1 die Titelverbindung hergestellt.

Die Ausgangsverbindungen werden wie folgt hergestellt:

(a) 4-(3-Cyanophenyl)-pyridin-N-oxid

Ausgehend von 4-(3-Cyanophenyl)-pyridin wird durch Oxidation mit $H_2O_2$ in Eisessig [J. Heterocycl. Chem. 11, 299 (1974)] die Titelverbindung hergestellt.

(b) 2-Cyano-4-(3-cyanophenyl)-pyridin

Ausgehend von 4-(3-Cyanophenyl)-pyridin-N-oxid wird analog Beispiel 2a die Titelverbindung hergestellt.

Beispiel 9: 4-Amidino-6-(3-amidinophenyl)-pyrimidin

Ausgehend von 4-Cyano-6-(3-cyanophenyl)-pyrimidin wird nach der Pinner-Methode zur Orthoester- und Amidinsynthese (z.B. 1. Umsetzung mit HCl-Gas und abs. Ethanol in Methylenchlorid; 2. Umsetzung mit Ammoniak) die Titelverbindung hergestellt.

Die Ausgangsverbindungen werden wie folgt hergestellt:

(a) 6-(3-Cyanophenyl)-4-hydroxy-pyrimidin

Ausgehend von 6-(3-Cyanophenyl)-4-hydroxy-2-methylthiopyrimidin [$\hat{=}$ 4-(3-Cyanophenyl)-6-hydroxy-2-methylthiopyrimidin, siehe Beispiel 10a] wird durch Desulfurierung mit Raney-Nickel in siedendem Ethanol die Titelverbindung erhalten.

(b) 4-Chlor-6-(3-cyanophenyl)-pyrimidin

6-(3-Cyanophenyl)-4-hydroxy-pyrimidin wird mit Phosphoroxychlorid am Rückfluss gekocht, wobei die Titelverbindung erhalten wird.

(c) 4-Cyano-6-(3-cyanophenyl)-pyrimidin

4-Chlor-6-(3-cyanophenyl)-pyrimidin wird mit Kupfercyanid in siedendem N,N-Dimethylformamid umgesetzt, wobei die Titelverbindung erhalten wird.

Beispiel 10: 2-Amidino-4-(3-amidinophenyl)-pyrimidin

Ausgehend von 2-Cyano-4-(3-cyanophenyl)-pyrimidin wird nach der Pinner-Methode zur Orthoester- und Amidinsynthese (z.B. 1 Umsetzung mit HCl-Gas und abs. Ethanol in Methylenchlorid; 2. Umsetzung mit Ammoniak) die Titelverbindung hergestellt.

Die Ausgangsverbindungen werden wie folgt hergestellt:

(a) 4-(3-Cyanophenyl)-6-hydroxy-2-methylthiopyrimidin

Eine Lösung von 1,1 g (0,02 Mol) Kaliumhydroxid in 5 ml dest. Wasser wird mit 4,3 g (0,02 Mol) 3-(3-Cyanophenyl)-3-oxo-propionsäureethylester und 2,2 g (0,02 Mol) S-Methylisothiuronium-iodid versetzt und 16 h bei Raumtemperatur gerührt. Das Reaktionsgemisch wird filtriert und das Filtrat mit Eisessig auf pH 4 gestellt. Das auskristallisierte Produkt wird abgesaugt, getrocknet und aus Methanol umkristallisiert. Man erhält so die Titelverbindung; Smp. $>220°$.

(b) 4-(3-Cyanophenyl)-6-chlor-2-methylthiopyrimidin

Eine Suspension von 4-(3-Cyanophenyl)-6-hydroxy-2-methylthiopyrimidin in Phosphoroxychlorid wird 5 h bei 110° gerührt. Das Reaktionsgemisch wird eingedampft, mit Eiswasser versetzt und durch Zugabe von 10 N Natronlauge auf pH 5-6 gestellt. Das ungelöste Material wird abgesaugt, mit Wasser gewaschen, im Vakuum getrocknet, und stellt die Titelverbindung dar. [Analogie zu J. pharm. Soc. Japan 70, 137 (1950)].

(c) 4-(3-Cyanophenyl)-2-methylthiopyrimidin

Ausgehend von 4-(3-Cyanophenyl)-6-chlor-2-methylthiopyrimidin wird analog J. pharm. Soc. Japan 70, 137 (1950) durch Reduktion mit Zink in Gegenwart von 5 %igem wässrigem Ammoniak die Titelverbindung erhalten.

(d) 4-(3-Cyanophenyl)-2-methylsulfonylpyrimidin

4-(3-Cyanophenyl)-2-methylthiopyrimidin wird in dest. Wasser suspendiert und analog Recl. Trav. Chim. (Pays-Bas) 93, 325 (1974) durch Durchleitung von Chlor zu der Titelverbindung umgesetzt.

(e) 2-Cyano-4-(3-cyanophenyl)-pyrimidin

Ein Gemisch von 4-(3-Cyanophenyl)-2-methylsulfonylpyrimidin und Kaliumcyanid in N,N-Dimethylformamid wird während 4 h bei 100° gerührt. Das Reaktionsgemisch wird eingedampft, mit dest. Wasser versetzt und mit Methylenchlorid extrahiert. Die organische Phase wird getrocknet und eingedampft, wobei die Titelverbindung erhalten wird.

Beispiel 11: 4,4′-Dithiocarbamoyl-2,2′-bipyridin

Eine Lösung von 2 g (0,01 Mol) 4,4′-Dicyano-2,2′-bipyridin und 2,74 ml (0,02 Mol) Triethylamin in 50 ml Pyridin wird mit Schwefelwasserstoff gesättigt, 5 h bei 40° und schliesslich 16 h bei Raumtemperatur gerührt. Das Reaktionsgemisch wird auf Wasser gegossen. Das ausgefallene Produkt wird abgesaugt, mit Wasser gewaschen und getrocknet. Man erhält so die Titelverbindung.

Beispiel 12: 6,6′-Dithiocarbamoyl-2,2′-bipyridin

Ausgehend von 6,6′-Dicyano-2,2′-bipyridin wird analog Beispiel 11 die Titelverbindung hergestellt.

Beispiel 13: Analog zu den Beispielen 1-3 werden folgende Verbindungen hergestellt:

a) 3,3′-Dicarbamoyl-biphenyl, Smp. 293-295°.
b) 4-Amidino-2-(3-amidinophenyl)-pyrimidin
c) 4-Amidino-2-(6-amidinopyrid-2-yl)-pyrimidin
d) 2-Amidino-4-(6-amidinopyrid-2-yl)-pyrimidin
e) 6,6′-Dicarbamoyl-2,2′-bipyridin
f) 6,6′-Bis-N-methylamidino-2,2′-bipyridin-dihydrochlorid, Smp. >300°, IR (KBr): 1670, 1621, 1563, 1420, 976, 800, 685 $cm^{-1}$.
g) 6,6′-Bis-1-piperidinoiminomethyl-2,2′-bipyridin
h) 6,6′-Bis-1-pyrrolidinoiminomethyl-2,2′-bipyridin-dihydrochlorid, Smp. >300°, IR (KBr): 1666, 1620, 1570, 1423, 990, 814 $cm^{-1}$.
i) 6,6′-Bis-N-hydroxyamidino-2,2′-bipyridin-dihydrochlorid, Smp. 280-284°.
j) 6,6′-Bis-N-aminoamidino-2,2′-bipyridin
k) 6,6′-Bis-N-cyclopentylamidino-2,2′-bipyridin-dihydrochlorid, Smp. >300°, IR (KBr) : 1669, 1630, 1442, 991, 753 $cm^{-1}$.
l) 6,6′-Bis-N,N-dimethylamidino-2,2′-bipyridin-dihydrochlorid, Smp. 310-312°, IR (KBr): 1674, 1580, 1428, 994, 821 $cm^{-1}$.

Beispiel 14: 3,3′-Dithiocarbamoyl-biphenyl

1,0 g (4,9 mMol) 3,3′-Dicyano-biphenyl werden in 22 ml Pyridin und 1,4 ml (9,8 mMol) Triethylamin gelöst. In die gelbe Lösung leitet man während 7 h und bei 40° trockenen Schwefelwasserstoff ein. Die grüne Reaktionslösung wird weitere 15 h bei 40° gerührt, abgekühlt und in Wasser gegossen. Man extrahiert mit Methylenchlorid, trocknet die organische Phase mit Natriumsulfat, dampft ein und kristallisiert die Titelverbindung aus wenig Methylenchlorid um; Smp. 188-190°.

Beispiel 15: 4,4′-Bis-N-n-propylcarbamoyl-2,2′-bipyridin

Eine Lösung von 0,258 g (10,5 mMol) 2,2′-Bipyridin-4,4′-dicarbonsäure in 10 ml Dimethylformamid und 0,29 ml Triethylamin wird unter Rühren mit 0,535 g (21 mMol) 2-Ethyl-5-phenyl-isoxazolium-3′-sulfonat (Woodwards Reagens K, Fa. Fluka) versetzt. Man rührt bis zur vollständigen Auflösung des letztgenannten Reagens (ca. 1 h) und versetzt die gelbe Lösung mit 0,18 ml n-Propylamin. Man rührt das Reaktionsgemisch weitere 5 h bei Raumtemperatur nach, saugt das ausgefallene Produkt ab und kristallisiert aus Ethanol um. Man erhält so die Titelverbindung als weisse Kristalle; Smp. 290-291°.

Beispiel 16: Kapseln enthaltend 0,25 g Wirkstoff, z.B. eine der Verbindungen der Beispiele 1-15, können wie folgt hergestellt werden:

Zusammensetzung (für 5000 Kapseln)

| | |
|---|---|
| Wirkstoff | 1250 g |
| Talk | 180 g |
| Weizenstärke | 120 g |
| Magnesiumstearat | 80 g |
| Laktose | 20 g |

Die pulverförmigen Substanzen werden durch ein Sieb mit einer Maschenweite von 0,6 mm getrieben und gemischt. Portionen von je 0,33 g des Gemisches werden mittels einer Kapselfüllmaschine in Gelatine-Kapseln abgefüllt.

**Patentansprüche**

1. Verbindungen der Formel I

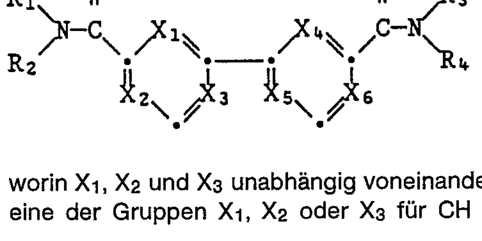

(I)

worin $X_1$, $X_2$ und $X_3$ unabhängig voneinander CH oder N bedeuten mit der Massgabe, dass mindestens eine der Gruppen $X_1$, $X_2$ oder $X_3$ für CH steht; $X_4$, $X_5$ und $X_6$ unabhängig voneinander CH oder N

bedeuten mit der Massgabe, dass mindestens eine der Gruppen $X_4$, $X_5$ oder $X_6$ für CH steht; Y für $NR_5$, O oder S steht, Z für $NR_6$, O oder S steht, die Reste $R_2$, $R_4$, $R_5$, und $R_6$ unabhängig voneinander Wasserstoff oder Niederalkyl bedeuten und die Reste $R_1$ und $R_3$ unabhängig voneinander für Wasserstoff, Niederalkyl, Cycloalkyl, Arylniederalkyl, Aryl, freies oder funktionell abgewandeltes Carboxy, Hydroxy, verethertes oder verestertes Hydroxy oder unsubstituiertes oder mono- oder disubstituiertes Amino stehen; worin die Reste $R_1$ und $R_2$ zusammen auch Niederalkylen bedeuten können, worin die Reste $R_3$ und $R_4$ zusammen auch für Niederalkylen stehen können, worin die Reste $R_2$ und $R_5$ zusammen auch Niederalkylen bedeuten können, und worin die Reste $R_4$ und $R_6$ zusammen auch für Niederalkylen stehen können;mit der Massgabe, dass Y für $NR_5$ oder S steht und Z $NR_6$ oder S bedeutet, wenn $X_3$ und $X_5$ für N und $X_1$, $X_2$, $X_4$ und $X_6$ für CH stehen;

und mit der Massgabe, dass Y $NR_5$ und Z $NR_6$ bedeuten, wenn

(a) $X_4$ und $X_5$ für N und $X_1$, $X_2$, $X_3$ und $X_6$ für CH stehen oder

(b) $X_1$ und $X_3$ für N und $X_2$, $X_4$, $X_5$ und $X_6$ für CH stehen;

Tautomere davon, und Salze davon.

2. Verbindungen der Formel I gemäss Anspruch 1, worin $X_1$, $X_2$ und $X_3$ unabhängig voneinander CH oder N bedeuten mit der Massgabe, dass mindestens eine der Gruppen $X_1$, $X_2$ oder $X_3$ für CH steht; $X_4$, $X_5$ und $X_6$ unabhängig voneinander CH oder N bedeuten mit der Massgabe, dass min destens eine der Gruppen $X_4$, $X_5$ oder $X_6$ für CH steht; Y für NH, O oder S steht, Z für NH, O oder S steht, die Reste $R_2$ und $R_4$ unabhängig voneinander Wasserstoff oder Niederalkyl bedeuten und die Reste $R_1$ und $R_3$ unabhängig voneinander für Wasserstoff, Niederalkyl, $C_3$-$C_8$-Cycloalkyl, Phenylniederalkyl, Phenyl, Carboxy, Hydroxy oder Amino stehen; worin die Reste $R_1$ und $R_2$ zusammen auch $C_2$-$C_7$-Alkylen bedeuten können, und worin die Reste $R_3$ und $R_4$ zusammen auch für $C_2$-$C_7$-Alkylen stehen können;

mit der Massgabe, dass Y und Z NH oder S bedeuten, wenn $X_3$ und $X_5$ für N und $X_1$, $X_2$, $X_4$ und $X_6$ für CH stehen;

und mit der Massgabe, dass Y und Z NH bedeuten, wenn

(a) $X_4$ und $X_5$ für N und $X_1$, $X_2$, $X_3$ und $X_6$ für CH stehen oder

(b) $X_1$ und $X_3$ für N und $X_2$, $X_4$, $X_5$ und $X_6$ für CH stehen;

Tautomere davon, und Salze davon.

3. Verbindungen der Formel I gemäss Anspruch 1, worin $X_1$, $X_2$ und $X_3$ unabhängig voneinander CH oder N bedeuten mit der Massgabe, dass mindestens eine der Gruppen $X_1$, $X_2$ oder $X_3$ für CH steht; $X_4$, $X_5$ und $X_6$ unabhängig voneinander CH oder N bedeuten mit der Massgabe, dass mindestens eine der Gruppen $X_4$, $X_5$ oder $X_6$ für CH steht; Y für NH steht, Z für NH steht, die Reste $R_2$ und $R_4$ unabhängig voneinander Wasserstoff oder Niederalkyl bedeuten und die Reste $R_1$ und $R_3$ unabhängig voneinander für Wasserstoff, Niederalkyl, $C_3$-$C_8$-Cycloalkyl, Phenylniederalkyl, Phenyl, Carboxy, Hydroxy oder Amino stehen; worin die Reste $R_1$ und $R_2$ zusammen auch $C_2$-$C_7$-Alkylen bedeuten können, und worin die Reste $R_3$ und $R_4$ zusammen auch für $C_2$-$C_7$-Alkylen stehen können; Tautomere davon, und Salze davon.

4. Verbindungen der Formel I gemäss Anspruch 1, worin (a) $X_1$ und $X_4$ für N stehen und $X_2$, $X_3$, $X_5$ und $X_6$ CH bedeuten oder (b) $X_2$ und $X_6$ für N stehen und $X_1$, $X_3$, $X_4$ und $X_5$ CH bedeuten; Y für NH oder O steht, Z für NH oder O steht, die Reste $R_2$ und $R_4$ Wasserstoff bedeuten und die Reste $R_1$ und $R_3$ für Wasserstoff, Niederalkyl, Hydroxy oder Amino stehen, Tautomere davon, und pharmazeutisch verwendbare Salze davon.

5. Verbindungen der Formel I gemäss Anspruch 1, worin (a) $X_1$ und $X_4$ für N stehen und $X_2$, $X_3$, $X_5$ und $X_6$ CH bedeuten oder (b) $X_2$ und $X_6$ für N stehen und $X_1$, $X_3$, $X_4$ und $X_5$ CH bedeuten; Y für NH steht, Z für NH steht, die Reste $R_2$ und $R_4$ Wasserstoff bedeuten und die Reste $R_1$ und $R_3$ für Wasserstoff, Niederalkyl, $C_5$-$C_6$-Cycloalkyl oder Hydroxy stehen, Tautomere davon, und pharmazeutisch verwendbare Salze davon.

6. Eine Verbindung der Formel I gemäss einem der Ansprüche 4 oder 5, worin $R_1$, $R_2$, $R_3$ und $R_4$ Wasserstoff bedeuten.

7. 2,2′-Diamidino-4,4′-bipyridin oder ein pharmazeutisch verwendbares Salz davon.

8. 6,6′-Diamidino-2,2′-bipyridin oder ein pharmazeutisch verwendbares Salz davon.

9. Pharmazeutisch Präparate enthaltend eine Verbindung gemäss einem der Ansprüche 1 bis 8 und mindestens ein pharmazeutisch verwendbares Trägermaterial.

10. Eine Verbindung gemäss einem der Ansprüche 1 bis 8 zur Anwendung in einem Verfahren zur therapeutischen Behandlung des tierischen oder menschlichen Körpers.

11. Eine Verbindung gemäss einem der Ansprüche 1 bis 8 zur Verwendung als antitumor-wirksames Mittel.

12. Verwendung von einer Verbindung gemäss einem der Ansprüche 1 bis 8 zur Herstellung pharmazeutischer Präparate.

13. Verwendung von einer Verbindung gemäss einem der Ansprüche 1 bis 8 zur Herstellung pharmazeutischer Präparate für die Behandlung von Tumoren.

14. Verfahren zur Herstellung einer Verbindung der Formel I gemäss Anspruch 1, dadurch gekennzeichnet, dass man in einer Verbindung der Formel II

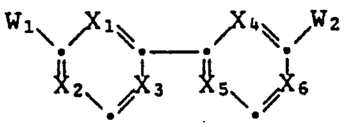

(II)

worin $W_1$ und $W_2$ jeweils einen in die Gruppe $-C(=Y)NR_1R_2$ bzw. $-C(=Z)NR_3R_4$ überführbaren Rest bedeuten, die Reste $W_1$ und $W_2$ in die Gruppe $-C(=Y)NR_1R_2$ bzw. $-C(=Z)NR_3R_4$ überführt; wobei die Symbole $X_1$-$X_6$, Y, Z und $R_1$-$R_4$ die unter Formel I angegebene Bedeutung haben; und/oder eine erhaltene Verbindung der Formel I in eine andere Verbindung der Formel I unwandelt, und/oder ein erhaltenes Salz in die freie Verbindung oder in ein anderes Salz umwandelt, und/oder eine erhaltene freie Verbindung der Formel I mit salzbildenden Eigenschaften in ein Salz umwandelt.

Europäisches Patentamt

# EUROPÄISCHER RECHERCHENBERICHT

Nummer der Anmeldung

EP 89 81 0491

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | JOURNAL OF THE AMERICAN CHEMICAL SOCIETY Band 80, 1958, Seiten 2745-2748; G. MAERKER et al.: "The Synthesis of Some 4,4'-Disubstituted 2,2'-Bipyridines" * Seite 2747, linke Spalte, letzter Absatz * --- | 1,2,14 | C 07 D 213/78<br>C 07 D 213/81<br>C 07 D 213/83<br>C 07 D 239/28<br>C 07 C 233/07<br>C 07 C 257/18<br>C 07 C 327/38<br>C 07 D 401/04<br>A 61 K 31/44 |
| A | JOURNAL OF THE AMERICAN CHEMICAL SOCIETY Band 104, 1982, Seiten 7519-7526; C.M. ELLIOTT et al.: "Electrochemical and Spectral Investigations of Ring-Substituted Bipyridine Complexes of Ruthenium" * Seite 7519, rechte Spalte, vorletzter Absatz - Seite 7520, Abschnitt iv; Seite 7520, Figur 1, Verbindung 5 * --- | 1,2,14 | |
| Y | CHEMICAL ABSTRACTS Band 105, Nr. 9, 1. September 1986, Seite 16, Zusammenfassung Nr. 72085r, Columbus, Ohio, USA; I. ANTONINI et al.: "2,2'-Bipyridyl-6-carbothioamide derivatives as potential antitumor agents" & Farmaco, Ed. Sci. 1986, Band 41, Nr. 5, Seiten 346-354 --- | 1,11,13 | **RECHERCHIERTE SACHGEBIETE (Int. Cl.5)**<br>C 07 C 233/00<br>C 07 C 257/00<br>C 07 C 327/00<br>C 07 D 213/00<br>C 07 D 239/00<br>C 07 D 401/00 |
| Y | CHEMICAL ABSTRACTS Band 105, Nr. 11, 15. September 1986, Seite 30, Zusammenfassung Nr. 90903k, Columbus, Ohio, USA; G. CRISTALLI et al.: "2,2'-Bipyridyl-6-carboxamidoximes with potential antitumor and antimicrobial properties" & Farmaco, Ed. Sci. 1986, Band 41, Nr. 7, Seiten 499-507 --- -/- | 1,11,13 | |

Der vorliegende Recherchenbericht wurde für alle Patentansprüche erstellt

| Recherchenort | Abschlußdatum der Recherche | Prüfer |
|---|---|---|
| BERLIN | 15-09-1989 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

EP 89 81 0491

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,P | CHEMICAL ABSTRACTS Band 109, Nr. 26, 26. Dezember 1988, Seite 836, Zusammenfassung Nr. 242947a, Columbus, Ohio, USA; G. CRISTALLI et al.: "Metal(II) complexes of 2,2'-bipyridyl-6-carbothioamide as anti-tumor and anti-fungal agents" & Eur. J. Med. Chem. 1988, Band 23, Nr. 3, Seiten 301-305 | 1,11,13 | |
| A | CHEMICAL ABSTRACTS Band 88, Nr. 5, 30. Januar 1978, Seite 482, Zusammenfassung Nr. 37578n, Columbus, Ohio, USA; P. NANTKA-NAMIRSKI et al.: "Bipyridyls. IX. Synthesis and reactions of 2-hydroxy-6-pyridylnicotinie acid derivatives" & Acta Pol. Pharm. 1977, Band 34, Nr. 2, Seiten 133-138 | 1,11,13 | |
| A | US-A-4 598 073 (G. R. NEWKOME) * Ansprüche 1,12 * | 1,11,13 | RECHERCHIERTE SACHGEBIETE (Int. Cl.5) |

Der vorliegende Recherchenbericht wurde für alle Patentansprüche erstellt

| Recherchenort | Abschlußdatum der Recherche | Prüfer |
|---|---|---|
| BERLIN | 15-09-1989 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)